# EUROPÄISCHE PATENTSCHRIFT

(11) **EP 0 731 849 B1**
(45) Veröffentlichungstag und Bekanntmachung des Hinweises auf die Patenterteilung: **03.03.2004**
(21) Anmeldenummer: 95904500.6
(22) Anmeldetag: 17.12.1994
(51) Int. Cl.: C12Q 1/68

(54) **ANORDNUNG VON NUKLEINSÄURESEQUENZEN UND DEREN VERWENDUNG**
ARRANGEMENT OF NUCLEIC ACID SEQUENCES AND ITS USE
AGENCEMENT DE SEQUENCES D'ACIDES NUCLEIQUES ET SON UTILISATION

(30) Priorität: 27.12.1993 DE 4344726
(43) Veröffentlichungstag der Anmeldung: 18.09.1996
(60) Teilanmeldung: 02008759.9
(73) Patentinhaber: Deutsches Krebsforschungszentrum, 69120 Heidelberg (DE)
(72) Erfinder: CREMER, Thomas, D-69126 Heidelberg (DE); RIED, Thomas, D-69121 Heidelberg (DE); SPEICHER, Michael, D-69124 Heidelberg (DE); JAUCH, Anna, D-68542 Heddesheim (DE); LICHTER, Peter, D-69251 Gaiberg (DE)
(74) Vertreter: Geyer, Werner, Dr.-Ing.
(86) Internationale Anmeldenummer: PCT/EP1994/004208
(87) Internationale Veröffentlichungsnummer: WO 1995/018235

(56) Entgegenhaltungen:
- WO-A-93/18186
- WO-A-93/21345
- SEMINARS IN CANCER BIOLOGY, Bd.4, Nr.1, Februar 1993 Seiten 41 - 46 KALLIONIEMI, O-P. ET AL 'comparative genomic hybridization: a rapid new method for detecting and mapping DNA amplification in tumors'
- HUMAN GENETICS, Bd.90, 1993, BERLIN, GERMANY Seiten 590 - 610 MANOIR, S. ET AL 'Detection of complete and partial chromosome gains and losses by comparative genomic in situ hybridization'

## Beschreibung

Die Erfindung betrifft ein Verfahren sowie die Verwendung einer Anordnung zur vergleichenden Nucleinsäure-Hybridisierung.

Mit Verfahren der vergleichenden genomischen in situ Hybridisierung (comparative genomic hybridization, CGH) auf Referenz-Chromosomenpräparaten mit normalem Karyotyp können derzeit in einer genomischen Test-DNA (z. B. Tumor-DNA, genomische Probanden-DNA mit Verdacht auf unbalanzierte Chromosomenaberrationen) Gewinne und Verluste von Genomabschnitten von etwa 10 Mbp erfaßt werden. Bei Amplifikationen können auch wesentlich kleinere DNA-Abschnitte durch CGH auf Referenzchromosomenpräparate kartiert werden. Diese Verfahren sind aus Du Manoir, S., Speicher, M.R., Joos, S., Schröck, E., Popp, S., Döhner, H., Kovacs, G., Robert-Nicoud, M., Lichter, P., Cremer, T.: Detection of complete and partial chromosome gains and losses by comparative genomic in situ hybridiyzation. Hum. Genet. 90:590-610, 1993, oder Joos, S., Scherthan, H., Speicher, M.R., Schlegel, J., Cremer, T., Lichter, P.: Detection of amplified genomic sequences by reverse chromosome painting using genomic tumor DNA as probe. Hum. Genet. 90:584-589, 1993, bekannt. Als genomische Referenz-DNA kann DNA genommen werden, die - falls verfügbar - aus Zellen mit normalem Chromosomenkomplement derselben oder auch einer anderen Person gewonnen wird.

Der heute erreichte Stand der Entwicklung von CGH hat zwei wesentliche Limitierungen. Zum einen ist eine weitere Erhöhung des Auflösungsvermögens wünschenswert. Es ist zu erwarten, daß auf Prometaphasechromosomen CGH Analysen partieller Trisomien und Monosomien mit einem Auflösungsvermögen von etwa ≥ 3 Mbp möglich werden. Dies entspricht dem mittleren DNA-Gehalt einer Chromosomenbande bei einer hochauflösenden Chromosomenbänderung mit ca. 1000 Banden pro haploidem Chromosomensatz. Für viele Anwendungen wäre jedoch ein CGH-Test wünschenswert, mit dem auch Gewinne und Verluste einzelner Gene oder sogar intragenischer DNA-Abschnitte sicher erfaßt werden können. Möglicherweise lassen sich weitere Verbesserungen des Auflösungsvermögens erreichen, wenn CGH-Analysen auf noch stärker dekondensierten Chromatinstrukturen durchgeführt werden. Zum anderen haben CGH-Tests auf mitotischen Referenzchromosomen den Nachteil, daß die vollautomatische Identifizierung von Chromosomen nach Fluoreszenzbänderung, z. B. mit DAPI, und die Messung der CGH-Fluoreszenzquotienten-Profile entlang der einzelnen Chromosomen technisch aufwendig und zeitraubend ist.

Aufgabe der Erfindung ist es nun, ein Verfahren sowie die Verwendung einer Anordnung von Nukleinsäuresequenzen zur Verfügung zu stellen, mit der mit weniger technischem Aufwand eine Automatisierung und eine wesentlich höhere Auflösung erzielt werden kann.

Diese Aufgabe wird durch die Merkmale des Patentanspruchs 1 bzw. 13 gelöst. Die Unteransprüche beschreiben vorteilhafte Ausgestaltungen der Anordnung.

Eine entscheidende Verbesserung sowohl im. Hinblick auf das Auflösungsvermögen als auch im Hinblick auf eine vollautomatische Auswertung wird durch einen VNH-Matrix Test (VNH = Vergleichende Nukleinsäure-Hybridisierung) erreicht, bei dem anstelle mitotischer Chromosomen spezifische Nukleinsäuresequenzen (im folgenden als Ziel-Nukleinsäuren bezeichnet, im Falle von DNA als Ziel-DNA, im Falle von RNA als Ziel-RNA) auf einem geeigneten Träger-Material (im folgenden Matrix genannt) aufgebracht werden. Eine Ziel-Nukleinsäure kann aus einer oder vielen verschiedenen DNA- bzw. RNA-Sequenzen bestehen. Die Komplexität einer Ziel-Nukleinsäure richtet sich dabei nach der jeweiligen Fragestellung. Der VNH-Matrix Test soll eine vollautomatische Gewinn- und Verlust-Bilanz genetischer Imbalanzen in einer genomischen Test-DNA ermöglichen, bei der das Auflösungsvermögen für ausgewählte Genomabschnitte, z. B. einzelne Gene, im kbp-Bereich liegen kann.

Die Ziel-Nukleinsäuren werden auf einer festen Matrix immobilisiert, die beispielsweise aus Filter-Papier oder aus Glas besteht. Das Areal der Matrix, in dem eine Ziel-Nukleinsäure aufgebracht ist, wird im folgenden als Slot bezeichnet. Anschließend erfolgt die simultane Hybridisierung von Testund Referenz-DNA gegen die Ziel-Nukleinsäuren. Alternativ dazu kann die Hybridisierung von Test- und Referenz-DNA gegen die Ziel-Nukleinsäuren auch in Lösung vorgenommen werden. Dabei muß für jede Ziel-Nukleinsäure eine getrennte Hybridisierung durchgeführt werden. Die Auswertung erfolgt dann nach Bindung der Hybridisierungprodukte an eine solide Matrix oder direkt in Lösung.

Im Gegensatz zu der stark variablen Anordnung individueller Chromosomen in Metaphasespreitungen, wie sie bei einer vergleichenden genomischen in situ Hybridisierung eingesetzt werden, kann die Position der auf Gewinne und Verluste in der Test-DNA zu prüfenden Genomabschnitte auf einer Matrix eindeutig festgelegt werden. Weiterhin zeigen Größe und Form individueller Chromosomen von Metaphase zu Metaphase erhebliche Schwankungen, während Größe und Geometrie der einzelnen Slots normiert werden können. Diese Möglichkeiten einer Normierung von Position, Größe und Geometrie der Ziel-Nukleinsäuren-Slots erleichtern die vollautomatische Auswertung einer Matrix im Vergleich zu CGH auf Metaphasechromosomen entscheidend. Größe und Abstand der einzelnen Slots können so gewählt werden, daß eine automatische Steuerung eines Tisches mit der darauf aufgebrachten Matrix oder - alternativ - eines Lichtstrahls leicht mit genügender Präzision realisiert werden kann. Bei Bedarf können Fluoreszenzquotienten innerhalb eines Slots auch in mehreren getrennten Arealen bestimmt und daraus Mittelwerte ermittelt werden.

Die Erfindung wird im folgenden anhand eines VNH-Matrix Tests zur Analyse von Imbalancen genomischer DNA bzw. exprimierter RNA in verschiedenen Geweben und Zelltypen und anhand von sieben Beispielen näher erläutert.

Für die vergleichende Quantifizierung der Genexpression in verschiedenen Geweben und Zelltypen soll ein Test entwickelt werden, der auf der vergleichenden Hybridisierung von unterschiedlich markierter mRNA bzw. cDNA von zwei Geweben oder Zelltypen auf eine Matrix mit den entsprechenden cDNA-Klonen beruht.

Das Prinzip des VNH-Matrix Tests beruht auf der vergleichenden Hybridisierung von Test- und Referenz Nukleinsäure Proben gegen Ziel Proben, die auf Glas oder einem Filter aufgebracht wurden, und der quantitativen Bestimmung von Fluoreszenzquotienten für die hybridisierten Proben. Die einzelnen Verfahrensschritte werden im Folgenden dargestellt.

### 1. Auswahl von Test- und Referenz-DNA bzw. -RNA Proben

Die Auswahl von genomischen Test- und Referenz DNAs erfolgt nach denselben Kriterien wie bei CGH Tests auf Metaphasechromosomen. Neben gesamtgenomischer DNA kann auch mittels DOP-PCR amplifizierte genomische DNA verwendet werden. Dies ist z. B. bei Speicher, M.R., du Manoir, S., Schröck, E., Holtgreve-Grez, H., Schoell, B., Lengauer, C., Cremer, T., Ried, T.: Molecular cytogenetic analysis of formalin-fixed, paraffin-embedded solid tumors by comparative genomic hybridizationafter universal DNA-amplification. Hum. Mol. Genet. 2:1907-1914, 1993 beschrieben. Als Test- und Referenz-Proben für vergleichende Tests der Genexpression können mRNA Präparationen bzw. cDNA Bibliotheken ausgewählter Zellen bzw. Gewebe, aber auch einzelne cDNA-Proben und Kombinationen von cDNA-Proben eingesetzt werden.

### 2. Auswahl von Ziel-DNA bzw. Ziel-RNA

Als Ziel-Nukleinsäuren die in der unten beschriebenen Weise auf die Matrix aufgetragen werden, kommen klonierte genomische DNA-Abschnitte einer Spezies (z. B. Mensch) in Frage beispielsweise DNA-Präparationen von Plasmid-Klonen, Cosmid-Klonen, Pl-Klonen, YAC-Klonen, die Genomabschnitte von wenigen kbp bis zu mehreren Mbp umfassen. Statt gereinigter Nukleinsäuren können auch sortierte Chromosomen oder Mikroorganismen, die entsprechende Zielnukleinsäuren enthalten, direkt auf die Matrix aufgebracht werden.

Die physikalische Kartierung der verwendeten Proben sollte bekannt sein. Für noch größere Genomabschnitte, z.B. bestimmte Chromosomenbanden, Chromosomenarme, ganze Chromosomen können Mischungen der DNA ausgewählter genomischer DNA-Klone zusammengestellt oder es können DNAs von Klonbibliotheken verwendet werden, die von sortierten oder mikrodissezierten Chromosomen des Menschen oder anderer Spezies hergestellt wurden. Für vergleichende Tests der Genexpression können cDNA-Proben, Kombinationen von cDNA-Proben bzw. cDNA-Bibliotheken, sowie mRNA-Fraktionen als Ziel-Nukleinsäuren eingesetzt werden.

### Herstellung der VNH-Test-Matrix

Die für einen gewünschten VNH-Matrix-Test erforderlichen Ziel-Nukleinsäuren werden auf einem Filter in einer vom Experimentator gewünschten geometrischen Anordnung aufgebracht, beispielsweise so daß die Reihenfolge genomischer Ziel-Nukleinsäuren auf der Matrix von oben nach unten der Reihenfolge ihrer physikalischen Kartierung auf einem Chromosom von pter bis qter entspricht. Jeder Probe ist demnach ein Slot mit einer definierten Position auf dem Filter zugeordnet. Als Filter können für Nukleinsäure-Bindung übliche Papierfilter eingesetzt werden. Bei Fluoreszenz-Verfahren müssen die Filter so ausgewählt werden, daß ihre Eigenschaften - wie z. B. die Eigenfluoreszenz - die Messung der Fluoreszenzsignale nicht stören werden. Auf diese Weise können die Slots für verschiedene Chromosomen, Chromosomenabschnitte und Gene nebeneinander in parallelen Reihen angeordnet werden. Die Auswahl der Ziel-Nukleinsäuren hängt von der diagnostischen Zielsetzung und dem benötigten Auflösungsvermögen des VNH-Matrix-Tests ab. Eine Slot-Matrix kann Ziel-Nukleinsäuren für exprimierte Sequenzen oder genomische Abschnitte ausgewählter Gene, ebenso wie Ziel-DNAs für Chromosomenabschnitte, inidividuelle Chromosomen oder auch den gesamten Chromosomensatz beinhalten. Ihre Anzahl kann je nach diagnostischer Zielsetzung von einigen wenigen bis zu einigen hundert Ziel-Nukleinsäuren variieren. Bei den Ziel-Nukleinsäuren kann es sich um einzelsträngige Proben oder um doppelsträngige Proben handeln. Im letzteren Falle muß die Ziel-Nukleinsäure vor dem VNH-Test durch einen geeigneten Denaturierungsschritt einzelsträngig gemacht werden. Die Ziel-Nukleinsäuren müssen durch geeignete Behandlung der Filter so an die Filter gebunden werden, daß sie sich während der VNH-Prozedur nicht ablösen können.

Für die Herstellung der VNH-Test-Matrix auf Glas sind Verfahren notwendig, die die Bindung der Ziel-DNA bzw. Ziel-RNA auf Glas gewährleisten. Dafür existieren bereits verschiedene Protokolle, wie z.B. die Beschichtung von Objektträgern mit einem dünnen Polyacrylamid-Film und der anschließenden Immobilisierung der aufzutragenden Proben nach einem Verfahren von Khrapko et al. (Khrapko,K.R., Lysov, Y.P., Khorlin, A.A., Ivanov, I.B., Yershov, G.M., Vasilenko, S.K., Florentiev, V.L., Mirzabekov, A.D.: A method for DNA sequencing by hybridization with oligonucleotide matrix. DNA-Sequence-J. DNA-Sequencing and Mapping 1:375-388, 1991). Eine andere Möglichkeit besteht in der Beimischung von Trägersubstanzen - wie z.B. von Proteinen, die höchstens zu geringen oder unterscheidbaren Hintergrundsignalen führen - zu den Ziel-Nukleinsäuren, dem Auftrag des Gemischs auf die Matrix und einer anschließenden Fixierung wie z.B. mit Methanol/Eisessig oder Formaldehyd. Die Auswahl und Anordnung der Proben auf der Glasmatrix werden wie oben beschrieben durchgeführt. Statt Glas bieten sich auch andere harte Materialien an. Besonders geeignet erscheinen Mikroplatten mit vorgeformten Vertiefungen.

Als Alternative zur Herstellung einer VNH-Matrix auf Glas oder auf Filter kann die Hybridisierung auch - für jede Ziel-Nukleinsäure getrennt - in Lösung durchgeführt werden. Bei dieser Methode muß besonderer Wert auf die quantitative Abtrennung der nicht-hybridisierten Probenmoleküle gelegt werden. Dies kann mit konventionellen Methoden wie z. B. Gelfiltration, Gelelektrophorese, Chromatographie oder durch enzymatischen Abbau erfolgen. Erst nach dieser Abtrennung werden die Signalintensitäten der Test- und der Referenz-DNA gemessen. Diese Messung kann sowohl nach Bindung der Hybridisierungsprodukte an eine solide Matrix oder - für jede Ziel-Nukleinsäuren getrennt - in Lösung erfolgen. Die Messung nach Bindung an eine solide Matrix erfolgt wie unten ausgeführt, die Messung in Lösung kann stationär für jeden Reaktionsansatz oder in automatisierter Form, z.B. im Durchflußspektrophotometer, erfolgen. Dabei können die Signale von Test- und Referenz-Nukleinsäuren entsprechend der Signalcharakteristik gemessen werden. Beispielsweise können Test- und Referenz-Nukleinsäuren über verschiedene Fluorochrome markiert werden. Beide lassen sich dann nach dem Stand der Technik in der Fluorometrie getrennt anregen und messen und nach dem Stand der Technik in der Flußcytometrie simultan angeregen und getrennt messen.

Die Markierung der Nukleinsäure Proben mit Haptenen (z. B. Biotin oder Digoxigenin) oder direkt mit einem Fluorochrom erfolgt mittels molekulargenetischen Standardverfahren (z. B. Nick Translation, Random Priming) wie bei Lichter, P., Cremer, T.: Chromosome analysis by non-isotopic in situ hybridization. in: Human cytogenetics: A practical approach; eds.: Rooney, D.E., Czepulkowski, B.H., IRL Press, Oxford: 157-192, 1992, und Raap, A.K., Wiegant, J., Lichter, P.: Multiple fluorescence in situ hybridization for molecular cytogenetics. in: Techniques and Methods in Molecular Biology: Non-radioactive labeling and detection of biomolecules; ed.: Kessler, C., Springer Verlag, Berlin, Heidelberg, New York:343-354, 1992, beschrieben.

Die Durchführung der vergleichenden Nukleinsäure Hybridisierung erfolgt wie bei Du Manoir, S., Speicher, M.R., Joos, S., Schröck, E., Popp, S., Döhner, H., Kovacs, G., Robert-Nicoud, M., Lichter, P., Cremer, T.: Detection of complete and partial chromosome gains and losses by comparative genomic in situ hybridiyzation. Hum. Genet. 90:592-593, 1993, bzw. Speicher, M.R., du Manoir, S., Schröck, E., Holtgreve-Grez, H., Schoell, B., Lengauer, C., Cremer, T., Ried, T.: Molecular cytogenetic analysis of formalin-fixed, paraffin-embedded solid tumors by comparative genomic hybridizationafter universal DNA-amplification. Hum. Mol. Genet. 2:1913-1914, 1993, beschrieben. Die Hybridisierung von RNA-Proben erfolgt analog und unter Berücksichtigung der bei RNA-Hybridisierungen üblichen Kautelen.

Der Nachweis der hybridisierten Probensequenzen erfolgt über Moleküle, die quantitativ erfaßbare Signale hervorrufen, welche sich genügend von "Hintergrund"-Signalen auf der Matrix unterscheiden. Hierzu werden gegenwärtig Fluoreszenz-Eigenschaften bevorzugt. Bei Fluorochrom-markierten Nukleinsäuren erfolgt der Nachweis direkt nach der Durchführung der üblichen Waschschritte. Die Durchführung von Fluoreszenz-Nachweisreaktionen bei Hapten-markierten Nukleinsäure-Proben erfolgt nach Standardverfahren wie z.B. bei Lichter, P., Cremer, T.: Chromosome analysis by non-isotopic in situ hybridization. in: Human cytogenetics: A practical approach; eds.: Rooney, D.E., Czepulkowski, B.H., IRL Press, Oxford: 157-192, 1992, beschrieben. Neben der Fluorezenz können auch andere Nachweisverfahren angewandt werden, die zu gut quantifizierbaren Signalen führen, wie beispielsweise die Chemilumineszenz, Phosphoreszenz, Radioaktivität zum direkten oder indirekten Nachweis von Nukleinsäuren. Es können auch verschiedene Nachweisarten für Test- und Referenz-Nukleinsäuren in einem Experiment kombiniert werden.

Im Anschluß an die VNH-Prozedur werden die Fluoreszenzsignale für jeden Slot der Matrix quantitativ bestimmt (z. B. mit einer CCD-Kamera) und daraus der Fluoreszenzquotient Test Nukleinsäure/Referenz Nukleinsäure mit einem Mikroprozessor berechnet. Die Bestimmung der Fluoreszenzquotienten erfolgt wie bei du Manoir et al. (1993) (S. 592-593) bzw. Speicher et al. (1993) (S. 1913-1914) beschrieben mit dem Unterschied, daß die Messungen mit Hilfe von Masken nicht an einzelnen Chromosomen sondern innerhalb der einzelnen Ziel-Nukleinsäuren Slots erfolgt. In VNH-Kontrollexperimenten mit unterschiedlich markierten genomischen DNAs von Zellen mit normalem Karyotyp bzw. unterschiedlich markierter identischer cDNA oder RNA Proben werden die auch normalerweise zu erwartenden Schwankungen dieser Quotienten auf einem vorgegebenen Vertrauensniveau bestimmt. Bei Probanden mit einer durch den Test erfaßbaren genomischen Duplikation oder Deletion eines Chromosoms, eines Chromosomenabschnitts oder eines Gens ist eine systematische Erhöhung bzw. Erniedrigung der Quotienten in den Slots zu erwarten, die die entsprechenden Ziel-Nukleinsäuren enthalten. Die Fluoreszenz-Quotienten für die übrigen Slots sollten dagegen im Kontrollbereich liegen.

Da das vom Testgenom herrührende Hybridisierungssignal in jedem Slot mit dem von der normalen Referenz-DNA herrührenden Hybridisierungssignal verglichen wird, sollte der VNH Matrix Test relativ unempfindlich gegenüber Schwankungen der Ziel-Nukleinsäuren Menge in den einzelnen Slots bei der Produktion der Matrix sein. Interexperimentelle Schwankungen im Mischungsverhältnis der Tumor DNA und Referenz DNA wirken sich auf alle Quotienten gleichsinnig aus und lassen sich ebenfalls leicht normieren.

Ein wichtiger Aspekt betrifft die Auswahl geeigneter Ausrüstung zur quantitativen Erfassung der Hybridisierungssignale. Die Nachweisinstrumente müssen generell in der Lage sein, lineare Unterschiede der Signalintensitäten über einen weiten Bereich zu messen. Zum Nachweis von Fluoreszenz-Signalen bieten sich gegenwärtig verschiedene Instrument-Konfigurationen an, wie z. B.: Fluoreszenzmikroskope, die mit einer (gekühlten) CCD (charged coupled device) Kamera ausgerüstet sind; oder Fluoro-Scanner, bei denen ein Fluoreszenz-Scanning über einen elektronisch gesteuerten Laserstrahl und die Detektion über einen sensitiven Photomultiplier erfolgt. Auch bei der Durchflußspektophotometrie erfolgt die Anregung über eine Lampe oder einen Laser und der Nachweis über Photomultiplier.

Je nach Art der Nachweissignale (s. o.) sind auch andere Verfahren, wie z. B. Densitometrie (siehe z. B. Phopshor-Imaging), angebracht.

Alle Meßdaten sollen in digitaler Form erfaßt und gespeichert werden. Die Verhältnisse der Signalintensitäten von Test- und Referenz-Nukleinsäuren werden dann mithilfe geeigneter Software errechnet.

### Anwendungsbeispiele

Wichtige Anwendungen liegen auf dem Gebiet der klinischen Genetik, Tumordiagnostik, der klinischen Pathologie, der Analyse von Tiermodellen für genetische Erkrankungen einschließlich Tumoren sowie der Züchtungsforschung.

Die Auswahl von Ziel-Nukleinsäuren für die Matrix erfolgt nach den diagnostischen Anforderungen. Sind für eine bestimmte diagnostische Problemstellung die in Frage kommenden chromosomalen Imbalancen bekannt, so kann eine Matrix mit Ziel-Nukleinsäuren hergestellt werden, die selektiv für den Nachweis bzw. den Ausschluß dieser spezifischen Imbalancen ausgewählt werden (siehe unten Beispiel 3). Für andere Fragestellungen ist es jedoch wünschenswert, eine möglichst umfassende Analyse des Genoms auf unbekannte Imbalancen durchzuführen. Dies erfolgt beispielsweise durch Aufspaltung eines gesamten Genoms in eine Reihe von Ziel-Nukleinsäuren. Das Auflösungsvermögen und die Sensitivität eines solchen VNH Tests werden dabei von der Anzahl und der genomischen Verteilung der Ziel-Nukleinsäuren bestimmt (siehe unten Beispiel 2). Um beispielsweise das Auflösungsvermögen einer cytogenetischen Bänderungsanalyse mit 400 bzw. 800 Chromosomen-Banden pro haploidem Chromosomensatz zu erreichen, müßte jede Bande auf der Matrix durch eine geeignete Ziel-Nukleinsäure repräsentiert sein, im folgenden "400 bzw. 800 Banden-Matrix" genannt (siehe Beispiel 2). Mit dieser Matrix könnte man Verluste und Gewinne von chromosomalen Regionen auf dem so vorgegebenen Auflösungsniveau bestimmen, das dem zur Zeit erreichbaren Auflösungsvermögen von CGH auf Metaphase-Chromosomen entspricht.

Bei Bedarf kann eine sequentielle Testung verschiedener Matrizen mit unterschiedlichem Auflösungsvermögen durchgeführt werden. Wenn beispielsweise auf normalen Chromosomen oder auf einer 400 Banden-Matrix der Gewinn oder Verlust eines bestimmten Chromosomen-Segments erkannt wird, kann in einem zweiten Schritt eine Matrix eingesetzt werden, mit deren Hilfe die Bruchpunkte der imbalancierten Region genauer eingegrenzt werden. Für diese Matrix werden Ziel-Nukleinsäuren verwendet, die definierte Subregionen des zuvor identifizerten Chromosomen-Segments charakterisieren (Beispiel 3).

### Beispiel 1:

Screening numerischer Chromosomen Aberrationen. Hierzu sind 24 Ziel-DNAs erforderlich, die die 24 verschiedenen menschlichen Chromosomen repräsentieren. Diese werden nach den diagnostischen Anforderungen zusammengestellt (siehe unten). Als Ziel-DNAs kommen in Frage: DNA sortierter menschlicher Chromosomen; DNA von somatischen Hybridzellen, die jeweils ein menschliches Chromosom enthalten (monochromosomale Hybridzellen); DNA-Amplifikationsprodukte von sortierten menschlichen Chromosomen oder monochromosomalen Hybridzellen; Pools aus klonierten, Chromosomen-spezifischen Fragmenten wie z.B. YACs, P1-Klone, Cosmide, oder entsprechend Contigs solcher Proben. Statt DNAs könnten auch sortierte Chromosomen oder Mikroorganismen, die entsprechende Zielnukleinsäuren enthalten, direkt auf die Matrix aufgebracht werden (siehe oben).

### Mögliche Anwendungen:

a) Pränatales Screening embryonaler Zellen nach numerischen Veränderungen. Die wichtigsten numerischen Veränderungen betreffen die Chromosomen 13, 18, 21, X und Y. Dementsprechend enthält die Matrix in diesem Fall die Ziel-DNAs der genannten 5 Chromosomen. Wenn aus ethischen und legalen Erwägungen ein Screening der Geschlechtschromosomen ausgeschlossen werden soll, würden nur Ziel-DNAs für die Chromosomen 13, 18, 21 aufgetragen.
b) Screenen nach Hyperploidien in Patienten mit akuter lymphatischer Leukämie, da Hyperploidien mit n>50 eine günstige klinische Prognose haben. In diesem Falle erscheint es sinnvoll, Ziel DNAs für alle 24 menschlichen Chromosomen aufzutragen.
c) Screenen von Tumoren, in denen numerische Aberrationen eine Rolle spielen, wie z.B. chromophobe Nierenzellkarzinome, Blasenkarzinome. Auch hier können Matrizen verwendet werden, die Ziel-DNAs aller 24 Chromosomen (dies scheint beim Blasenkarzinom angebracht) oder Ziel-DNAs der bei einzelnen Tumor-Entitäten relevanten Aberrationen enthalten (z. B. chromophobe Nierenzellkarzinome).

### Beispiel 2:

Universelles Screening von unbekannten partiellen Chromosomen-Imbalancen. Hierzu sind Ziel-DNAs erforderlich, die verschiedene Abschnitte der menschlichen Chromosomen repräsentieren. So können in Analogie zu den derzeitigen molekularbiologischen Methoden der Analyse von genomischen Verlusten ("loss of heterozygosity, LOH") Matrizen mit 42 Ziel-DNAs verwendet werden, um alle relevanten Chromosomenarme zu repräsentieren: 1p, 1q, 2p, 2q, 3p, 3q, 4p, 4q, 5p, 5q, 6p, 6q, 7p, 7q, 8p, 8q, 9p, 9q, 10p, 10q, 11p, 11q, 12p, 12q, 13q, 14q, 15q, 16p, 16q, 17p, 17q, 18p, 18q, 19p, 19q, 20p, 20q, 21q, 22q, Yq.

Bei höherem Auflösungsbedarf können komplexere Matrizen eingesetzt werden, wie die oben beschriebenen "400 oder 800 Banden Matrizen".

### Mögliche Anwendungen:

a) Screenen von Patienten mit Verdacht auf unbekannte strukturelle Chromosomen Aberrationen.
b) Screenen von beliebigen Tumoren auf unbekannte chromosomale Imbalancen. Dieser Ansatz hat vor allem in der tumorbiologischen Forschung Bedeutung, da für viele Tumoren die diagnostisch und prognostisch relevanten genomischen Imbalancen bislang nicht identifiziert wurden.

### Beispiel 3:

Hochauflösendes Screening von bestimmten Chromosomen-Abschnitten auf genomische Imbalancen. In diesem Fall werden Matrizen hergestellt, die Ziel-DNAs nur für ausgewählte Chromosomen-Abschnitte haben und einer spezifischen diagnostischen Fragestellung Rechnung tragen.

Mögliche Anwendungen:
a) Bei einer genetischen Beratung von Familien mit reziproken Translokationen ist es wichtig zu wissen, ob es im Bereich der chromosomalen Bruchpunkte zu genetischen Imbalancen gekommen ist. Für eine derartige Analyse kann eine hochauflösende Matrix mit Ziel-DNAs hergestellt werden, die in den fraglichen Bruchpunktregionen kartiert sind.
b) Bei einer Carrier-Diagnostik von X-chromosomal rezessiven Erkrankungen, wie der Duchenne'schen Muskeldystrophie, kann eine Matrix hergestellt werden, die Ziel-DNAs für Abschnitte des entsprechenden Gens enthält.

### Beispiel 4:

Screenen nach genomischen Imbalancen von Tumor-relevanten Genen. Hierzu sind Ziel-DNAs erforderlich, die bekannte Proto-Onkogene, Tumorsuppressorgene oder andere für Wachstum und Metastasierung relevante Gene eines Tumors repräsentieren.

### Mögliche Anwendungen:

a) Nachweis der Amplifikation von Onkogenen mit prognostischer Relevanz, z.B. N-myc Amplifikation beim Neuroblastom.
b) Nachweis der Deletion von Tumorsuppressorgenen mit prognostischer Relevanz, z.B. die Deletion in 1p36 beim Neuroblastom.

### Beispiel 5:

Screenen auf Über- bzw. Unterexpression bestimmter Gene. Hierzu sind Ziel-Nukleinsäuren erforderlich, die kodierende Sequenzen ausgewählter Gene enthalten. Hierfür neben den in Beispiel 4 beschriebenen Matrizen auch Matrizen mit RNAs oder cDNAs der Gene in Frage. Als Test-Nukleinsäure wird Gesamt-RNA aus einer zu testenden Zell-Population isoliert, als Referenz-Nukleinsäure dient die Gesamt-RNA einer geeigneten Kontroll-Zell-Population mit normaler Expression der relevanten Gene.

### Mögliche Anwendung:

Bei einer genomischen Amplifikation von N-myc (siehe Beispiel 4a) kann mit diesem Test eine quantitative Erfassung der tatsächlichen Überexpression erfolgen.

### Beispiel 6:

Die oben für menschliche Erkrankungen aufgeführten Beispiele lassen sich in analoger Weise auf Tiermodelle für diese Erkrankungen ausdehnen. Voraussetzung dafür ist die Herstellung von Matrizen, deren Ziel-Nukleinsäuren von der entsprecehneden Spezies stammen oder eine für die Zwecke eines VNH-Tests genügende evolutionäre Konservierung aufweisen.

### Mögliche Anwendung:

Bei vielen Tiermodellen für spezifische Tumore ist zunächst nicht bekannt, ob der zu Grunde liegende genetische Mechanismus mit dem beim Menschen auftretenden Tumor übereinstimmt. In diesem Falle ist bei einer Testung von Tumor-relevanten Genen (siehe Beispiel 4) oder bei Expressionsanalyse (siehe Beispiel 5) eine Übereinstimmuing der Ergebnisse der VNH-Tests beim menschlichen und tierischen Tumor zu erwarten.

### Beispiel 7:

Bei einer Herstellung transgener Organismen können VNH-Tests mit Matrizen entwickelt werden, die Ziel-Nukleinsäuren der übertragenen Gene enthalten. Mit diesen Tests können die Kopienzahlen der übertragenen Gene und die Expression im Empfänger-Organismus quantitativ bestimmt werden.

### Mögliche Anwendungen:

a) Analyse von transgenen Tieren, mit entsprechend mutierten Tumor-relevanten Genen.
b) Züchtung von Tieren und Pflanzen mit veränderten Eigenschaften.

## Patentansprüche

1. Verfahren zum Nachweis der relativen Kopienzahl von Nukleinsäure-Sequenzen in Test-Zellen durch vergleichende Nukleinsäure-Hybridisierung eines Gemischs von markierten Test-Nukleinsäuren mit einem Gemisch von unterschiedlich markierten Nukleinsäuren aus Referenz-Zellen (Referenz-Nukleinsäuren) gegen Ziel-Nukleinsäuren, so daß die relative Kopienzahl einzelner Nukleinsäuren innerhalb der Test-Nukleinsäuren im Vergleich zu Referenz-Nukleinsäuren getrennt mit Hilfe der Markierungen gemessen werden kann, wobei die Ziel-Nukleinsäuren auf einem Träger geeigneten Materials in eindeutig festgelegter Weise getrennt angeordnet werden, **dadurch gekennzeichnet, daß** die Ziel-Nukleinsäuren in vorbestimmten Arealen auf dem Träger aufgebracht werden.

2. Verfahren nach Anspruch 1, **dadurch gekennzeichnet, daß** die Ziel-Nukleinsäure aus einer oder vielen verschiedenen DNA- bzw. RNA-Sequenzen bestehen.

3. Verfahren nach einem der vorhergehenden Ansprüche, **dadurch gekennzeichnet, daß** als Ziel-Nukleinsäure sortierte Chromosomen oder mikrodissezierte Chromosomenabschnitte oder klonierte genomische DNA-Abschnitte einer Spezies oder DNA-Bibliotheken von Chromosomen oder Chromosomen-Abschnitten oder Kombinationen von cDNA-Proben bzw. cDNA-Bibliotheken oder mRNA-Fraktionen einzeln oder in geeigneten Kombinationen eingesetzt werden.

4. Verfahren nach einem der vorhergehenden Ansprüche, **dadurch gekennzeichnet, daß** als Träger für die Ziel-Nukleinsäuren Glas oder Filtermaterial oder Gele oder Kunststoff oder Mikroplatten mit vorgeformten Vertiefungen verwendet werden.

5. Verfahren nach einem der vorhergehenden Ansprüche, **dadurch gekennzeichnet, daß** die Areale für Chromosomen, Chromosomen-Abschnitte und Gene nebeneinander in parallelen Reihen angeordnet werden.

6. Verfahren nach einem der vorhergehenden Ansprüche, **dadurch gekennzeichnet, daß** die Test- und Referenz-Nukleinsäuren mit Molekülen markiert sind, die quantitativ erfaßbare Signale hervorrufen, die sich genügend von Hintergrundsignalen auf dem Träger unterscheiden.

7. Verfahren nach Anspruch 6, **dadurch gekennzeichnet, daß** das Molekül ein Fluorochrom ist.

8. Verfahren nach Anspruch 7, **dadurch gekennzeichnet, daß** Fluoreszenzquotienten der Fluorochrome quantitativ bestimmt werden.

9. Verfahren nach Anspruch 8, **dadurch gekennzeichnet, daß** der Fluoreszenzquotient innerhalb eines Areals in mehreren getrennten Bereichen bestimmt und daraus der Mittelwert ermittelt wird.

10. Verfahren nach einem der Ansprüche 7 bis 9, **dadurch gekennzeichnet, daß** der Nachweis von Fluoreszenz-Signalen mit einem Fluoreszenzmikroskop erfolgt, das mit einer CCD-Kamera ausgerüstet ist.

11. Verfahren nach einem der Ansprüche 7 bis 9, **dadurch gekennzeichnet, daß** der Nachweis von Fluoreszenz-Signalen mit einem Fluoro-Scanner erfolgt, bei dem ein Fluoreszenz-Scanning über einen elektronisch gesteuerten Laserstrahl und die Detektion über einen sensitiven Photomultiplier erfolgt.

12. Verfahren nach einem der vorhergehenden Ansprüche, **dadurch gekennzeichnet, daß** Test- und Referenz-Nukleinsäuren ausgewählt sind unter gesamtgenomischer DNA, mittels DOP-PCR amplifizierter genomischer DNA, mRNA-Präparationen bzw. cDNA-Bibliotheken ausgewählter Zellen bzw. Gewebe, cDNA-Proben und Kombinationen von cDNA-Proben.

13. Verwendung einer Anordnung von Ziel-Nukleinsäuresequenzen auf einem Träger, wobei die Ziel-Nukleinsäuresequenzen in vorbestimmten Arealen auf dem Träger aufgebracht sind, zur vergleichenden Nukleinsäure-Hybridisierung nach einem der Ansprüche 1 bis 12.

14. Verwendung nach Anspruch 13, **dadurch gekennzeichnet, daß** als Träger für die Ziel-Nukleinsäuren Glas oder Filtermaterial oder Gele oder Kunststoff oder Mikroplatten mit vorgeformten Vertiefungen verwendet werden.

15. Verwendung nach Anspruch 13 oder 14, **dadurch gekennzeichnet, daß** als Ziel-Nukleinsäuren sortierte Chromosomen oder mikrodissezierte Chromosomenabschnitte oder klonierte genomische DNA-Abschnitte einer Spezies oder DNA-Bibliotheken von Chromosomen oder Chromosomen-Abschnitten oder Kombinationen von cDNA-Proben bzw. cDNA-Bibliotheken oder mRNA-Fraktionen einzeln oder in geeigneten Kombinationen eingesetzt werden.

16. Verwendung nach einem der Ansprüche 13 bis 15, **dadurch gekennzeichnet, daß** die Ziel-Nukleinsäuren auf einem mit einem dünnen Polyacrylamid-Film beschichteten Objektträger immobilisiert sind.

17. Verwendung nach einem der Ansprüche 13 bis 16, **dadurch gekennzeichnet, daß** als Ziel-Nukleinsäuren genomische Ziel-Nukleinsäuren verwendet werden, die auf dem Träger so aufgebracht sind, daß die Reihenfolge genomischer Ziel-Nukleinsäuen auf dem Träger von oben nach unten der Reihenfolge ihrer physikalischen Kartierung auf einem Chromosom von pter bis qter entspricht.

18. Verwendung nach einem der Ansprüche 13 bis 16, **dadurch gekennzeichnet, daß** die Areale für Chromosomen, Chromosomenabschnitte und Gene nebeneinander in parallelen Reihen angeordnet sind.

19. Verwendung nach einem der Ansprüche 13 bis 16, **dadurch gekennzeichnet, daß** der Träger 24 Ziel-Nukleinsäuren enthält, welche die 24 verschiedenen menschlichen Chromosomen repräsentieren.

20. Verwendung nach einem der Ansprüche 13 bis 16, **dadurch gekennzeichnet, daß** der Träger als Ziel-Nukleinsäure die Chromosomen 13,18, 21, X und Y enthält.

21. Verwendung nach einem der Ansprüche 13 bis 16, **dadurch gekennzeichnet, daß** der Träger 42 Ziel-Nukleinsäuren enthält, welche die relevanten Chromosomenarme repräsentieren: 1p, 1q, 2p, 2q, 3p, 3q, 4p, 4q, 5p, 5q, 6p, 6q, 7p, 7q, 8p, 8q, 9p, 9q, 10p, 10q, 11p, 11q, 12p, 12q, 13q, 14q, 15q, 16p, 16q, 17p, 17q, 18p, 18q, 19p, 19q, 20p, 20q, 21q, 22q, Yq.

22. Verwendung nach einem der Ansprüche 13 bis 16, **dadurch gekennzeichnet, daß** zur Erreichung des Auflösungsvermögens einer cytogenetischen Bänderungsanalyse mit 400 bzw. 800 Chromosomen-Banden pro haploidem Chromosomensatz jede Bande auf dem Träger durch eine geeignete Ziel-Nukleinsäure repräsentiert ist.

## Claims

1. Method of detecting the relative copy number of nucleic acid sequences in test cells by the comparative nucleic acid hybridisation of a mixture of labelled test nucleic acids with a mixture of variously labeled nucleic acids from reference cells (reference nucleic acids) against target nucleic acids, so that the relative copy number of individual nucleic acids within the test nucleic acids can be separately measured in comparison with reference nucleic acids with the aid of the labels, the target nucleic acids being separately arranged in a clearly stipulated manner on a support of suitable material, **characterised in that** the target nucleic acids are applied to predetermined areas of the support.

2. Method according to Claim 1, **characterised in that** the target nucleic acid consists of one or many different DNA/RNA sequences.

3. Method according to one of the preceding claims, **characterised in that** sorted chromosomes or microdissected chromosome segments, or cloned genomic DNA segments of a species, or DNA libraries of chromosomes or chromosome segments, or combinations of cDNA samples/cDNA libraries or mRNA fractions are used individually or in suitable combinations as the target nucleic acid.

4. Method according to one of the preceding claims, **characterised in that** glass, filter material, gels, plastic or microplates with preformed wells are used as the support for the target nucleic acids.

5. Method according to one of the preceding claims, **characterised in that** the areas for chromosomes, chromosome segments and genes are arranged next to one another in parallel rows.

6. Method according to one of the preceding claims, **characterised in that** the test and reference nucleic acids are labelled with molecules that emit quantitatively detectable signals that differ sufficiently from background signals on the support.

7. Method according to Claim 6, **characterised in that** the molecule is a fluorochrome.

8. Method according to Claim 7, **characterised in that** fluorescence quotients of the fluorochromes are determined quantitatively.

9. Method according to Claim 8, **characterised in that** the fluorescence quotient within an area is determined in several separate regions and their mean is calculated.

10. Method according to one of Claims 7 to 9, **characterised in that** fluorescence signals are detected using a fluorescence microscope equipped with a CCD camera.

11. Method according to one of Claims 7 to 9, **characterised in that** fluorescence signals are detected using a fluoro-scanner in which fluorescence scanning takes place by means of an electronically controlled laser beam and detection takes place by means of a sensitive photomultiplier.

12. Method according to one of the preceding claims, **characterised in that** test and reference nucleic acids are chosen from total genomic DNA, genomic DNA amplified by DOP-PCR, mRNA preparations/cDNA libraries of selected cells/tissue, cDNA samples and combinations of cDNA samples.

13. Use of an arrangement of target nucleic acid sequences on a support, the target nucleic acid sequences being applied to predetermined areas of the support, for comparative nucleic acid hybridisation according to one of Claims 1 to 12.

14. Use according to Claim 13, **characterised in that** glass, filter material, gels, plastic or microplates with preformed wells are used as the support for the target nucleic acids.

15. Use according to Claim 13 or 14, **characterised in that** sorted chromosomes or microdissected chromosome segments, or cloned genomic DNA segments of a species, or DNA libraries of chromosomes or chromosome segments, or combinations of cDNA samples/cDNA libraries or mRNA fractions are used individually or in suitable combinations as the target nucleic acids.

16. Use according to one of Claims 13 to 15, **characterised in that** the target nucleic acids are immobilized on an object support coated with a thin polyacrylamide film.

17. Use according to one of Claims 13 to 16, **characterised in that** the target nucleic acids used are genomic target nucleic acids applied to the support in such a way that the order of genomic target nucleic acids on the support from top to bottom corresponds to the order of their physical mapping on a chromosome from p ter to q ter.

18. Use according to one of Claims 13 to 16, **characterised in that** the areas for chromosomes, chromosome segments and genes are arranged next to one another in parallel rows.

19. Use according to one of Claims 13 to 16, **characterised in that** the support contains 24 target nucleic acids representing the 24 different human chromosomes.

20. Use according to one of Claims 13 to 16, **characterised in that** the support contains chromosomes 13, 18, 21, X and Y as the target nucleic acid.

21. Use according to one of Claims 13 to 16, **characterised in that** the support contains 42 target nucleic acids representing the relevant chromosome arms: 1p, 1q, 2p, 2q, 3p, 3q, 4p, 4q, 5p, 5q, 6p, 6q, 7p, 7q, 8p, 8q, 9p, 9q, 10p, 10q, 11p, 11q, 12p, 12q, 13q, 14q, 15q, 16p, 16q, 17p, 17q, 18p, 18q, 19p, 19q, 20p, 20q, 21q, 22q, Yq.

22. Use according to one of Claims 13 to 16, **characterised in that**, to achieve the resolution capability of a cytogenetic banding analysis with 400/800 chromosome bands per haploid set of chromosomes, each band on the support is represented by a suitable target nucleic acid.

## Revendications

1. Procédé de mise en évidence du nombre relatif de copies de séquences d'acides nucléiques dans des cellules tests par hybridation comparative des acides nucléiques d'un mélange d'acides nucléiques tests marqués avec un mélange d'acides nucléiques marqués différemment issus de cellules de référence (acides nucléiques de référence), par rapport à des acides nucléiques cibles, de telle sorte que le nombre relatif de copies de chacun des acides nucléiques figurant parmi les acides nucléiques tests puisse être mesuré séparément en comparaison des acides nucléiques de référence à l'aide des marquages, dans lequel les acides nucléiques cibles sont disposés séparément d'une façon définie sans équivoque sur un support de matériau approprié, **caractérisé en ce que** l'on applique les acides nucléiques cibles sur le support sur des aires prédéterminées.

2. Procédé selon la revendication 1, **caractérisé en ce que** les acides nucléiques cibles se composent d'une séquence ou de plusieurs séquences diverses d'ADN ou d'ARN.

3. Procédé selon l'une quelconque des revendications précédentes, **caractérisé en ce que** sont utilisés comme acides nucléiques cibles, individuellement ou sous la forme d'associations appropriées, des chromosomes classés, des segments de chromosomes microdisséqués, des segments clonés d'ADN génomique d'une espèce, des bibliothèques d'ADN de chromosomes ou de segments de chromosomes, des associations d'échantillons d'ADNc ou de bibliothèques d'ADNc, ou des fractions d'ARNm.

4. Procédé selon l'une quelconque des revendications précédentes, **caractérisé en ce qu'**on utilise comme support pour les acides nucléiques cibles du verre, un matériau filtrant, des gels, de la matière plastique ou des microplaques à puits préformés.

5. Procédé selon l'une quelconque des revendications précédentes, **caractérisé en ce que** les aires pour les chromosomes, les segments de chromosomes et les gènes sont disposées les unes à côté des autres en rangées parallèles.

6. Procédé selon l'une quelconque des revendications précédentes, **caractérisé en ce que** les acides nucléiques tests et de référence sont marqués avec des molécules donnant naissance à des signaux quantitativement détectables qui se distinguent suffisamment bien des signaux de fond sur le support.

7. Procédé selon la revendication 6, **caractérisé en ce que** la molécule est un fluorochrome.

8. Procédé selon la revendication 7, **caractérisé en ce que** l'on détermine les quotients de fluorescence des fluorochromes quantitativement.

9. Procédé selon la revendication 8, **caractérisé en ce que** le quotient de fluorescence à l'intérieur d'une aire est déterminé au niveau de plusieurs secteurs séparés et qu'on calcule la moyenne des résultats ainsi obtenus.

10. Procédé selon l'une quelconque des revendications 7 à 9, **caractérisé en ce que** la mise en évidence des signaux de fluorescence s'effectue avec un microscope à fluorescence équipé d'une caméra CCD.

11. Procédé selon l'une quelconque des revendications 7 à 9, **caractérisé en ce que** la mise en évidence des signaux de fluorescence s'effectue avec un fluoro-scanner faisant intervenir un rayon laser à commande électronique aux fins de réalisation d'un balayage de fluorescence et, pour la détection, un photomultiplicateur sensible.

12. Procédé selon l'une quelconque des revendications précédentes, **caractérisé en ce que** le choix des acides nucléiques tests et de référence s'opère entre un ADN génomique total, un ADN génomique amplifié par DOP-PCR, des préparations d'ARNm ou des bibliothèques d'ADNc de cellules ou de tissus sélectionnés, des échantillons d'ADNc ainsi que des associations d'échantillons d'ADNc.

13. Emploi d'une disposition de séquences d'acides nucléiques cibles sur un support, dans lequel les séquences d'acides nucléiques cibles sont appliquées sur le support sur des aires prédéterminées à des fins d'hybridation comparative d'acides nucléiques selon l'une quelconque des revendications 1 à 12.

14. Emploi selon la revendication 13, **caractérisé en ce qu'**on utilise comme support pour les acides nucléiques cibles du verre, un matériau filtrant, des gels, de 1a matière plastique ou des microplaques à puits préformés.

15. Emploi selon la revendication 13 ou 14, **caractérisé en ce que** sont utilisés comme acides nucléiques cibles, individuellement ou sous la forme d'associations appropriées, des chromosomes classés, des segments de chromosomes microdisséqués, des segments clonés d'ADN génomique d'une espèce, des bibliothèques d'ADN de chromosomes ou de segments de chromosomes, des associations d'échantillons d'ADNc ou de bibliothèques d'ADNc, ou des fractions d'ARNm.

16. Emploi selon l'une quelconque des revendications 13 à 15, **caractérisé en ce que** les acides nucléiques cibles sont immobilisés sur une lame porte-objets recouverte d'un mince film de polyacrylamide.

17. Emploi selon l'une quelconque des revendications 13 à 16, **caractérisé en ce que** sont employés comme acides nucléiques cibles des acides nucléiques cibles génomiques que l'on applique sur le support de manière à ce que la succession des acides nucléiques cibles génomiques sur le support corresponde, de haut en bas, à l'ordre de leur cartographie physique sur un chromosome de pter à qter.

18. Emploi selon l'une quelconque des revendications 13 à 16, **caractérisé en ce que** les aires pour les chromosomes, les segments de chromosomes et les gènes sont disposées les unes à côté des autres en rangées parallèles.

19. Emploi selon l'une quelconque des revendications 13 à 16, **caractérisé en ce que** le support contient 24 acides nucléiques cibles qui représentent les 24 différents chromosomes humains.

20. Emploi selon l'une quelconque des revendications 13 à 16, **caractérisé en ce que** le support contient comme acides nucléiques cibles les chromosomes 13, 18, 21, X et Y.

21. Emploi selon l'une quelconque des revendications 13 à 16, **caractérisé en ce que** le support contient 42 acides nucléiques cibles qui représentent les bras de chromosomes importants : 1p, 1q, 2p, 2q, 3p, 3q, 4p, 4q, 5p, 5q, 6p, 6q, 7p, 7q, 8p, 8q, 9p, 9q, 10p, 10q, 11p, 11q, 12p, 12q, 13q, 14q, 15q, 16p, 16q, 17p, 17q, 18p, 18q, 19p, 19q, 20p, 20q, 21q, 22q, Yq.

22. Emploi selon l'une quelconque des revendications 13 à 16, **caractérisé en ce qu'**aux fins de l'obtention du pouvoir séparateur d'une analyse de banding cytogénétique avec 400 ou 800 bandes de chromosomes par jeu de chromosomes haploïde, chaque bande est représentée sur le support par un acide nucléique cible approprié.
